# EUROPÄISCHE PATENTSCHRIFT

(11) **EP 2 630 931 B1**
(45) Veröffentlichungstag und Bekanntmachung des Hinweises auf die Patenterteilung: **25.11.2015**
(21) Anmeldenummer: 13151897.9
(22) Anmeldetag: 18.01.2013
(51) Int. Cl.: A61C 5/06, B05C 17/005

(54) **Abgabebehälter für Dentalmasse**
Dispensing container for dental composition
Récipient de distribution pour masse dentaire

(30) Priorität: 22.02.2012 DE 102012202693
(43) Veröffentlichungstag der Anmeldung: 28.08.2013
(73) Patentinhaber: Transcodent GmbH & Co. KG, 24149 Kiel (DE)
(72) Erfinder: Fritze, Joachim, 24211 Preetz (DE)
(74) Vertreter: Glawe, Delfs, Moll

(56) Entgegenhaltungen:
- EP-A2- 0 919 206
- US-A- 4 240 425
- US-A- 4 581 024
- US-A1- 2004 138 642

## Beschreibung

Die Erfindung betrifft ein Abgabesystem nach Anspruch 1 umfassend einen Abgabebehälter.

Solche Abgabebehälter werden dazu verwendet, Dentalmassen gezielt und dosiert auszubringen. Vor der Verwendung ist der Zustand des Abgabebehälters regelmäßig so, dass der Innenraum mit der Dentalmasse gefüllt ist und der Kolben in den Innenraum eingeführt ist, um den Innenraum abzuschließen und die Dentalmasse von der Umgebung zu trennen. Der Abgabebehälter wird dann mit einem Ausbringwerkzeug verbunden, so dass mit einem Stößel des Ausbringwerkzeugs Druck auf den Kolben ausgeübt werden kann. Unter dem Druck des Kolbens bewegt sich die Dentalmasse durch die Kanüle hindurch und tritt am vorderen Ende der Kanüle aus. Die Dentalmasse kann an Stellen ausgebracht werden, die ansonsten nur schwer zugänglich sind.

Bei der Herstellung des Abgabebehälters wird die Kanüle zunächst als separates Teil gefertigt und dann mit dem Gehäuse des Abgabebehälters verbunden. Beim Verbinden der Kanüle mit dem Abgabebehälter ist zu beachten, dass die Verbindung hinreichend fest ist, so dass die Verbindung sich auch dann nicht löst, wenn die Dentalmasse in dem Behälter unter Druck gesetzt wird. Außerdem muss die Verbindung dicht sein, so dass die Dentalmasse nur durch die Kanüle, nicht aber seitlich davon austreten kann.

Erreicht werden kann dies beispielsweise dadurch, dass das Gehäuse als Spritzgussteil hergestellt wird und die Kanüle direkt beim Spritzvorgang in das Gehäuse eingespritzt wird. Es bildet sich dann eine innige Verbindung zwischen dem Material des Gehäuses und der Außenwand der Kanüle, die den obigen Anforderungen genügt. Ein Nachteil besteht aber darin, dass eine exakt an das Gehäuse und die Kanüle angepasste Spritzgussform benötigt wird. Soll eine Kanüle mit größerem Durchmesser mit dem Gehäuse verbunden werden, so wird ein komplett neues Spritzgusswerkzeug benötigt.

Aus der EP 0 919 206 A2 ist eine Kartusche für dentale Anwendungen bekannt, die eine Kappe mit einer Kanüle aus Metall umfasst.

Der Erfindung liegt die Aufgabe zu Grunde, einen Abgabebehälter für Dentalmasse vorzustellen, der flexibel und kostengünstig hergestellt werden kann. Ausgehend vom eingangs genannten Stand der Technik wird die Aufgabe gelöst mit den Merkmalen des Anspruchs 1. Vorteilhafte Ausführungsformen finden sich in den Unteransprüchen.

Erfindungsgemäß besteht zwischen dem Umfang der Kanüle und dem Austrittskanal ein Spalt, in den ein Klebstoff eingebracht ist.

Zunächst werden einige Begriffe erläutert. Der Innenraum des Gehäuses ist für die Aufnahme von Dentalmasse bestimmt. Der Begriff Gehäuse, besagt nicht, dass der Innenraum rundherum abgeschlossen wäre. Regelmäßig ist außer dem Austrittskanal eine weitere Öffnung vorhanden, die mit einem Kolben verschlossen werden kann und über die die Dentalmasse unter Druck gesetzt werden kann. Die Richtung, in die die Kanüle aus dem Austritts kanal herausragt, wird als vorderes Ende, die entgegengesetzte Richtung als hinteres Ende bezeichnet. Der Begriff Dentalmasse soll nicht als Einschränkung im Hinblick auf die Konsistenz des Materials verstanden werden. Die Dentalmasse kann beispielsweise flüssig oder pastös sein.

Um die Kanüle mit dem Gehäuse zu verbinden, wird ein Abschnitt der Kanüle in den Austrittskanal des Gehäuses eingeführt. Der Klebstoff in dem zwischen der Kanüle und dem Austrittskanal verbleibenden Spalt stellt eine feste Verbindung zwischen der Kanüle und dem Gehäuse her. Diese Art der Herstellung ist kostengünstig und führt bei Verwendung eines geeigneten Klebstoffs zu einer sehr stabilen Verbindung zwischen Kanüle und Gehäuse. Die Stabilität der Verbindung ist gewünscht, weil bei der Verwendung des erfindungsgemäßen Abgabebehälters unterschiedliche Kräfte wie Zugkräfte, Druckkräfte, Biegekräfte und Drehmomente auf die Verbindung wirken können.

Der Spalt kann sich als Ringspalt rund um den Kanülenabschnitt herum erstrecken. Dabei kann der Spaltdurchmesser über den Umfang konstant sein. Möglich ist auch, dass die Spaltbreite über den Umfang variabel ist. Beispielsweise können zwei Abschnitte des Spalts durch einen Steg voneinander getrennt sein, der sich parallel zur Kanüle erstreckt. Der Steg kann auf der Außenfläche der Kanüle aufliegen oder es kann ein Spalt mit geringerem Durchmesser zwischen dem Steg und der Außenfläche bestehen.

Das Gehäuse ist vorzugsweise so gestaltet, dass in Längsrichtung eine eindeutige Position für die Kanüle definiert ist. Es kann dazu in dem Austrittskanal ein Anschlag vorgesehen sein, an dem die Kanüle anliegt, wenn sie die richtige Position in dem Austrittskanal hat. Der Anschlag kann von der Wand des Austrittskanals nach innen vorspringen, so dass die Stirnfläche der Kanüle an dem Anschlag anliegt. Indem die Kanüle bis zum Anschlag in den Austrittskanal eingeführt wird, ist es leicht, die richtige Position für die Kanüle zu finden. Der Anschlag kann so dimensioniert sein, dass er für Kanülen unterschiedlichen Durchmessers geeignet ist.

Wenn der Spalt sich über den gesamten Umfang der Kanüle und die gesamte Länge des in den Austrittskanal eingeführten Kanülenabschnitts erstreckt, hat die Kanüle zunächst einen Bewegungsspielraum in seitlicher Richtung. Erst durch den Klebstoff im Spalt wird die Kanüle in ihrer endgültigen Position fixiert. Von Vorteil kann es sein, wenn der Austrittskanal so gestaltet ist, dass er der Kanüle eine Seitenführung bietet, so dass die Position in seitlicher Richtung und die Ausrichtung der Kanüle eindeutig definiert sind. Dazu kann beispielsweise eine Mehrzahl von nach innen vorspringenden Stegen in dem Austrittskanal vorgesehen sein, die auf der Außenfläche der Kanüle aufliegen.

Als Seitenführung kann der Austrittskanal auch einen ersten Kanalabschnitt aufweisen, in dem die Innenfläche des Kanals an die Außenfläche der Kanüle angepasst ist. In dem ersten Kanalabschnitt kann beispielsweise ein flächiger Kontakt zwischen der Kanüle und dem Kanal bestehen, der sich über den gesamten Umfang der Kanüle erstreckt. Der Spalt besteht dann in erster Linie in einem zweiten Kanalabschnitt.

Vorzugsweise ist der erste Kanalabschnitt am hinteren Ende des Austrittskanals angeordnet. Der zweite Kanalabschnitt schließt sich nach vorne an den ersten Kanalabschnitt an. Der Klebstoff kann dann vom vorderen Ende des Austrittskanals in den Spalt des zweiten Kanalabschnitts eingebracht werden.

Für die Befüllung des Spalts mit Klebstoff ist es von Vorteil, wenn der Austrittskanal sich vom vorderen Ende in Richtung des hinteren Endes verjüngt, der Spaltdurchmesser sich also in dieser Richtung vermindert. Nicht ausgeschlossen ist es, dass die Verjüngung sich über die gesamte Länge des Austrittskanals erstreckt. Häufig umfasst die Verjüngung aber nur einen Abschnitt des Austrittskanals. Dies kann beispielsweise der zweite Kanalabschnitt sein. Um eine gleichmäßige Verteilung des Klebstoffes in dem Spalt zu fördern, können in dem sich verjüngenden Kanalabschnitt Vertiefungen ausgebildet sein. Im Bereich der Vertiefungen hat der Spalt einen etwas vergrößerten Durchmesser, so dass der Klebstoff leichter in Richtung des hinteren Endes des Austrittskanals vordringen kann. Der Klebstoff ist vorzugsweise heißpolymerisierend oder autopolymerisierend, damit ein Aushärten ohne Einfluss von Licht möglich ist. Um die Verklebung zu optimieren, können die Oberfläche der Kanüle und/oder der Austrittskanal vorkonditioniert werden. Für metallische Oberflächen kommen beispielsweise Sandstrahlen oder ein aufrauhender Ätzprozess in Betracht. Die Vorbehandlung von Kunststoffoberflächen kann durch thermische Behandlung, eine Koronaentladung oder eine andere oxidative Vorbehandlung erfolgen.

Möglich ist es, dass die Verjüngung sich im Wesentlichen gleichmäßig über den betreffenden Kanalabschnitt erstreckt. Alternativ kann die Verjüngung am vorderen Ende des Austrittskanals stärker ausgeprägt sein, so dass der Austrittskanal eine Mündungsaufweitung aufweist. Im Bereich der Mündungsaufweitung ist der Spaltdurchmesser vergrößert, so dass der Klebstoff leicht eingeführt werden kann.

Der die Kanüle umgebende Spalt kann sich über die gesamte Länge des in den Austrittskanal eingeführten Kanülenabschnitts erstrecken. Dies hat zur Folge, dass die Kanüle in dem Austrittskanal zunächst keiner eindeutigen Führung unterliegt, sondern in seitlicher Richtung bewegt werden kann. Bevor der Klebstoff in den Spalt eingeführt werden kann, muss durch geeignete Mittel sichergestellt werden, dass die Kanüle richtig im Austrittskanal zentriert ist. Erst wenn der Spalt mit dem Klebstoff gefüllt und der Klebstoff ausgehärtet ist, hat die Kanüle eine definierte Position in dem Austrittskanal. Die heute zur Verfügung stehenden Klebstoffe sind geeignet, einen Spalt auszufüllen und zugleich eine sehr stabile Verbindung herzustellen. Diese Art der Verbindungsherstellung hat den Vorteil, dass ein einheitlich dimensionierter Austrittskanal mit Kanülen unterschiedlichen Durchmessers versehen werden kann. So kann beispielsweise für Dentalmassen höherer Viskosität eine vergleichsweise kurze Kanüle mit großem Durchmesser verwendet werden. Eine längere Kanüle mit kleinem Durchmesser kann verwendet werden, damit die Dentalmasse beim Durchtritt durch die Kanüle bestimmten Scherkräften ausgesetzt wird. Der Abgabebehälter kann dadurch für unterschiedliche Verwendungszwecke angepasst werden, ohne dass an dem eigentlichen Gehäuse oder Austrittskanal etwas geändert werden müsste.

Bei dem Gehäuse mit Austrittskanal handelt es sich vorzugsweise um ein einstückiges Bauteil, das beispielsweise als Spritzgussteil kostengünstig hergestellt werden kann. Das Gehäuse ist vorzugsweise so bemessen, dass die Menge der aufgenommenen Dentalmasse für genau eine Anwendung bestimmt ist. Verglichen mit Abgabebehältern, die mehrere Anwendungseinheiten der Dentalmasse aufnehmen können, ist die Gefahr von Infektionen vermindert, weil keine Erreger von einem ersten zu einem zweiten Patienten übertragen werden können. Der anwendungsbereite Abgabebehälter ist mit Dentalmasse gefüllt, und ein Kolben ist in den Innenraum des Abgabebehälters eingeführt, so dass der Innenraum verschlossen ist.

Die Kanüle hat regelmäßig eine lang gestreckte zylindrische Form, deren Außendurchmesser beispielsweise zwischen 0,5 mm und 2 mm liegen kann. Im Inneren der Kanüle erstreckt sich ein Kanal, durch den die Dentalmasse hindurchtreten kann. Die Kanüle kann aus Metall gefertigt sein. Da beim Verbinden der Kanüle mit dem Gehäuse keine hohen Kräfte auf die Kanüle ausgeübt werden, kann die Kanüle aus einem weichgeglühten Stahl oder einem vergleichbaren Material bestehen. Dies hat den Vorteil, dass die Kanüle biegbar ist, also umgebogen werden kann, ohne dass sie bricht oder dass der Kanal im Inneren verschlossen wird. Der Anwender kann die Kanüle bei der Anwendung so formen, wie es ihm geeignet erscheint. Die Länge der Kanüle kann beispielsweise zwischen 13 mm und 23 mm liegen, damit die Kanüle tief in den Wurzelkanal eines Zahns eingeführt werden kann.

Durch größere Kanülenlängen von beispielsweise mindestens 15 mm, vorzugsweise von mindestens 20 mm können die Scherkräfte erhöht werden, die beim Durchtritt durch die Kanüle auf die Dentalmasse wirken. Bei einigen Dentalmassen sind solche Scherkräfte erwünscht, weil die Eigenschaften des Materials dadurch positiv beeinflusst werden.

Die Kanüle kann an ihrer vorderen Stirnfläche mit einer Austrittsöffnung versehen sein, so dass die Dentalmasse nach vorne ausgetragen werden kann. Zusätzlich oder alternativ dazu kann die Kanüle eine oder mehrere in seitlicher Richtung weisende Austrittsöffnungen aufweisen. Dies ermöglicht eine bessere Befüllung der lateralen Verzweigungen des Wurzelkanals.

Eine stabile Verbindung zwischen der Kanüle und dem Austrittskanal ist erforderlich, weil in verschiedenen Situationen hohe Kräfte auf die Kanüle wirken können. Wird eine Dentalmasse hoher Viskosität durch die Kanüle hindurchgezwungen, wirkt eine starke Kraft in Längsrichtung der Kanüle. Wird die Kanüle in seitlicher Richtung umgebogen, wirken starke Biegekräfte. Stößt die Spitze der umgebogenen Kanüle beim Einführen in den Zahn in seitlicher Richtung an, kann bezogen auf die Achse des Austrittskanals ein erhebliches Drehmoment entstehen. Es hat sich gezeigt, dass die erfindungsgemäße Klebeverbindung gegenüber allen diesen Belastungen hinreichende Stabilität bietet.

Das Gehäuse des erfindungsgemäßen Abgabebehälters kann eine Ausbringspitze aufweisen, in deren Innerem sich der Austrittskanal erstreckt. Die Außenfläche der Ausbringspitze kann so gestaltet sein, dass eine Kappe auf die Ausbringspitze aufgesteckt werden kann, die das vordere Ende des Abgabebehälters abdichtet. Beispielsweise kann die Ausbringspitze im Querschnitt rund sein und sich zum vorderen Ende hin leicht verjüngen. Der Abgabebehälter kann außerdem eine an die Ausbringspitze angepasste Kappe umfassen. Die Kappe ist vorzugsweise so bemessen, dass sie die Kanüle vollständig in sich einschließt. Im aufgesteckten Zustand kann die innere Stirnfläche der Kappe auf dem vorderen Ende der Kanüle aufliegen, um eine Austrittsöffnung der Kanüle zu verschließen. Für einen sicheren Verschluss kann an der inneren Stirnfläche der Kappe ein Vorsprung ausgebildet sein, der beispielsweise halbkugelförmig oder konusförmig sein kann.

Die Innenwand der Kappe kann am hinteren Ende nach außen gewölbt sein. Die Kapsel kann dann zum Umbiegen der Kanüle verwendet werden, indem die Wölbung als Widerlager an der Biegestelle angelegt wird und auf das vordere Ende der Kappe eine Kraft in seitlicher Richtung ausgeübt wird. Es wird dadurch eine gleichmäßige Kraftübertragung auf die Kanüle erreicht und verhindert, dass die Kanüle Schaden nimmt. Das Risiko einer Beschädigung der Kanüle wäre größer, wenn statt der Wölbung eine scharfe Kante der Kappe an der Kanüle anliegen würde.

Die Kappe soll so gestaltet sein, dass sie einerseits leicht auf die Ausbringspitze aufgesteckt werden kann und andererseits sicher mit der Ausbringspitze abdichtet. Auf der Innenwand der Kappe kann dazu ein umlaufender Steg ausgebildet sein, der bei aufgesteckter Kappe über den gesamten Umfang auf der Ausbringspitze aufliegt. Es kann eine Mehrzahl solcher Stege vorgesehen sein, die in Axialrichtung hintereinander angeordnet sind.

Um die Handhabung der Kappe zu erleichtern, können auf der Außenseite der Kappe Rippen vorgesehen sein, die sich vorzugsweise in Längsrichtung erstrecken. Der Anwender, der die Kappe aufstecken oder abziehen möchte, findet an den Rippen mit seinen Fingern guten Halt.

Die Kappe kann mit einem Anschlag versehen sein, der an dem Abgabebehälter zur Anlage kommt, sobald die Kanüle im richtigen Winkel umgebogen ist. Der betreffende Winkel zwischen dem vorderen Abschnitt der Kanüle und der Längsrichtung des Behälters wird so gewählt, dass das vordere Ende der Kanüle gut in eine Kavität eines Backenzahns eingeführt werden kann. Beispielsweise kann der Winkel zwischen 25° und 60°, vorzugsweise zwischen 30° und 45° liegen. Durch einen solchen Anschlag wird es dem Anwender erleichtert, die Kanüle im gewünschten Winkel umzubiegen.

Ein Winkel zwischen der Richtung der Kanüle und der Längsachse des Abgabebehälters kann auch dadurch erreicht werden, dass der Austrittskanal mit der Längsachse des Abgabebehälters einen Winkel einschließt. Auch dieser Winkel liegt vorzugsweise zwischen 25° und 60°, vorzugsweise zwischen 30° und 45°. In diesem Fall kann sich die Kanüle geradlinig in Verlängerung des Austrittskanals erstrecken und trotzdem gut in eine Kavität eines Backenzahns eingeführt werden.

Die Kanüle kann auch aus einem Material bestehen, das eine elastische Flexibilität aufweist. Die Kanüle kann sich dann beim Einführen in den Wurzelkanal so verformen, dass sie Biegungen des Wurzelkanals folgen kann. Es ist dann leichter, die Dentalmasse bis in die Spitze des Wurzelkanals auszubringen. Das Material der Kanüle kann eine Titanlegierung sein, vorzugsweise eine β-Titanlegierung. Es gibt bekanntlich β-Titanlegierungen mit einem sehr niedrigen Elastizitätsmodul. In einer alternativen Ausführungsform kann die Kanüle aus Polypropylen bestehen, das vorzugsweise lichtdicht eingefärbt ist. Auch dieses Material hat die gewünschte Flexibilität.

Die Erfindung betrifft außerdem ein Verfahren zum Herstellen eines Abgabebehälters für Dentalmasse. Bei dem Verfahren werden eine Kanüle und ein Gehäuse bereitgestellt. An der Innenseite des Gehäuses ist eine Gleitfläche ausgebildet, die zum Zusammenwirken mit einem Kolben bestimmt ist. Das Gehäuse umschließt einen Innenraum und ist mit einem Austrittskanal versehen. Die Kanüle wird mit einem Kanülenabschnitt in den Austrittskanal eingeführt, so dass zwischen dem Umfang des Kanülenabschnitts und dem Austrittskanal ein Spalt bleibt. In den Spalt wird ein Klebstoff eingebracht. Das Verfahren kann mit weiteren Merkmalen fortgebildet werden, die mit Bezug auf den erfindungsgemäßen Abgabebehälter beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine Draufsicht auf einen erfindungsgemäßen Abgabebehälter;
- Fig. 2:: eine Schnittansicht des Abgabebehälters aus Fig. 1;
- Fig. 3:: den Ausschnitt B des Gehäuses aus Fig. 2 in vergrößerter Darstellung;
- Fig. 4:: die Ansicht aus Fig. 3 bei anderen Ausführungsformen der Erfindung;
- Fig. 5:: einen erfindungsgemäßen Abgabebehälter mit aufgesetzter Kappe;
- Fig. 6:: die Kappe aus Fig. 5 in vergrößerter Darstellung;
- Fig. 7:: die Verwendung der Kappe beim Umbiegen der Kanüle; und
- Fig. 8:: eine weitere Ausführungsform eines erfindungsgemäßen Abgabebehälters.

Ein erfindungsgemäßer Abgabebehälter umfasst ein Gehäuse 14, das einen Innenraum 15 umschließt. Der Innenraum 15 ist zylinderförmig und am hinteren Ende 16 offen. Am vorderen Ende hat das Gehäuse 14 eine Ausbringspitze 17 mit einem Austrittskanal 18 im Inneren. In Verlängerung der Ausbringspitze 17 erstreckt sich eine Kanüle 19.

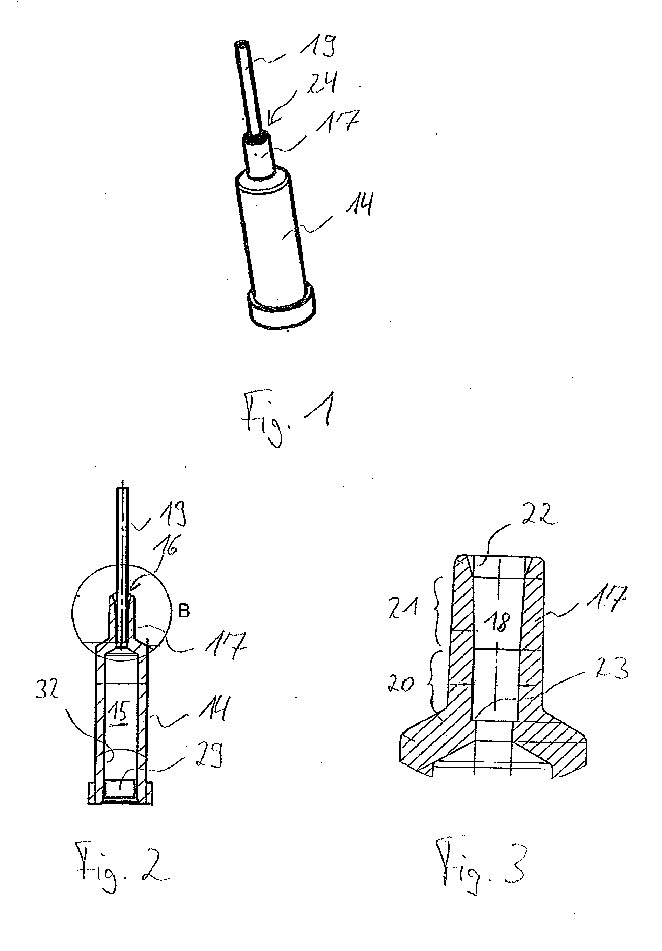

Im anwendungsbereiten Zustand ist der Innenraum 15 des Abgabebehälters mit einer Dentalmasse gefüllt, und das offene Ende des Innenraums 15 ist mit einem in Fig. 2 dargestellten Kolben 29 abgeschlossen. Der Kolben 29 wird durch die Innenseite des Gehäuses geführt, die als Gleitfläche 32 wird. Wenn über den Kolben 29 Druck auf die Dentalmasse ausgeübt wird, bewegt sich die Dentalmasse durch die Kanüle 19 hindurch und tritt am vorderen Ende der Kanüle aus. Da die Kanüle 19 lang und dünn ist, kann die Dentalmasse auch an solchen Stellen präzise ausgebracht werden, die ansonsten schwer zugänglich sind. Um Druck auf den Kolben 29 auszuüben, ist ein nicht dargestelltes Ausbringwerkzeug vorgesehen, das einen Stößel aufweist, um den Kolben 29 nach vorne zu drücken.

Der Austrittskanal 18 des Gehäuses 14 umfasst gemäß Fig. 3 einen ersten Kanalabschnitt 20 und einen zweiten Kanalabschnitt 21. Im ersten Kanalabschnitt 21 ist die Innenfläche des Austrittskanals 18 an die Außenfläche der Kanüle 19 angepasst. Die Kanüle 19 lässt sich nur dann in den ersten Kanalabschnitt 21 einführen, wenn die Kanüle 19 zentriert und richtig ausgerichtet ist. Am hinteren Ende des ersten Kanalabschnitts 21 ist ein Anschlag 23 ausgebildet, an den die Kanüle 19 anstößt, wenn sie ihre Endposition erreicht hat. Im ersten Kanalabschnitt 21 besteht zwischen der Außenwand der Kanüle 19 und der Innenwand des Austrittskanals 18 allenfalls ein geringfügiger Spalt, so dass die Kanüle 19 gerade ohne großen Widerstand eingesteckt werden kann.

In einem zweiten Kanalabschnitt 21 verjüngt sich der Durchmesser des Austrittskanals 18 in Richtung des hinteren Endes. Am hinteren Ende stimmt der Durchmesser mit dem ersten Kanalabschnitt 20 überein, so dass ein nahtloser Übergang zwischen den Kanalabschnitten 20, 21 besteht. An den zweiten Kanalabschnitt 21 schließt sich eine Mündungsaufweitung 22 an, durch die sich der Kanaldurchmesser zur Mündung hin deutlich vergrößert.

Die Kanüle 19 hat eine Länge von ungefähr 20 mm und der Austrittskanal 18 eine Länge von ungefähr 6 mm. Die Kanüle 19 ragt also um 14 mm gegenüber der Ausbringspitze 17 nach vorne. Der Durchmesser der Kanüle 19 kann je nach Ausführungsform zwischen 0,5 mm und 1,8 mm liegen. Die Kanüle 19 besteht aus einem weichgeglühten Stahl. Die Kanüle 19 kann also zur Seite umgebogen werden, ohne dass sie bricht oder der Kanal im Inneren verschlossen wird.

Wenn die Kanüle 19 ihre endgültige Position in dem Austrittskanal hat, bleibt im Bereich der Mündungsaufweitung 22 und des zweiten Kanalabschnitts 21 ein Spalt 16 zwischen der Außenwand der Kanüle 19 und dem Austrittskanal 18. Mit einem geeigneten Werkzeug wird ein Klebstoff 24 in den Bereich der Mündungsaufweitung 22 eingespritzt, so dass der Klebstoff entlang dem Spalt 16 nach hinten vordringt. Dabei wird so viel Klebstoff 24 eingebracht, dass der Spalt 16 vollständig mit Klebstoff 24 gefüllt ist. Es ist nicht ausgeschlossen, dass ein Teil des Klebstoffes 16 dabei auch in den ersten Kanalabschnitt 20 eindringt. Wenn der Klebstoff 16 ausgehärtet ist und eine feste Verbindung mit der Kanüle 19 und dem Austrittskanal 18 eingegangen ist, ist die Kanüle 19 sicher in dem Austrittskanal 18 fixiert.

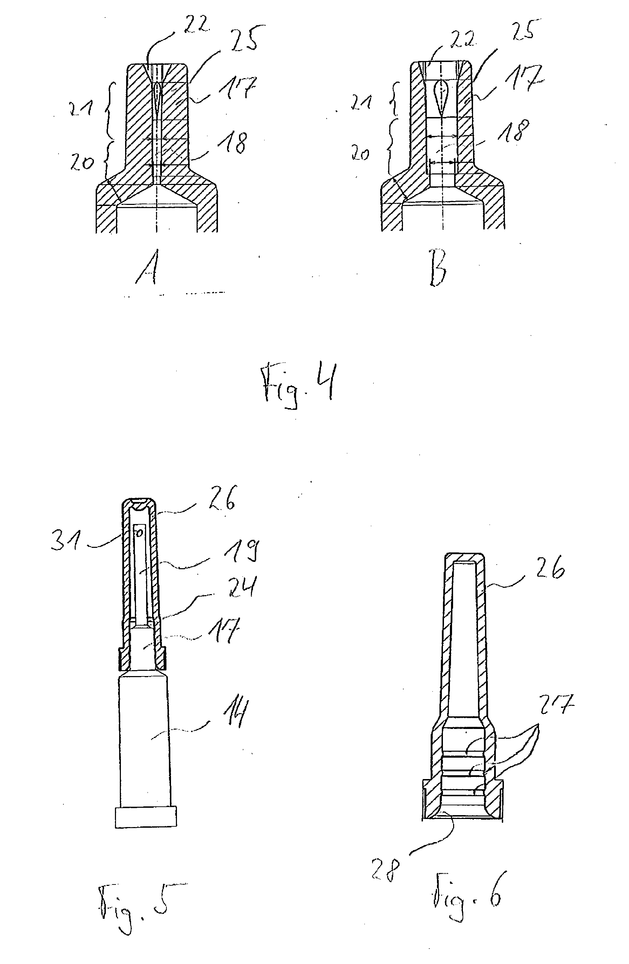

Die Fig. 4 zeigt in der linken Darstellung A eine Ausbringspitze 17, bei der der Austrittskanal 18 einen kleineren Durchmesser hat, und in der rechten Darstellung B eine Ausbringspitze 17, bei der der Austrittskanal einen größeren Durchmesser hat. Es ist ein wesentlicher Vorteil der Erfindung, dass eine einzige Spritzgussform ausreicht, um beide Ausführungsformen gemäß Fig. 4A und Fig. 4B herzustellen. Es muss dazu lediglich ein anderer Einsatz, der die Dimension des Austrittskanals 18 definiert, in die Spritzgussform eingesetzt werden.

Alternativ kann auch eine Kanüle 19 in den Austrittskanal 18 eingesetzt werden, die über die gesamte Länge einen kleineren Durchmesser hat als der Austrittskanal 18. Die Kanüle 19 wird erst dadurch fixiert, dass der Klebstoff den Spalt ausfüllt und aushärtet. Bei dieser Ausführungsform kann das Gehäuse mit dem Austrittskanal 18 als einheitliches Spritzgussteil gefertigt werden und je nach Anwendungsfall mit Kanülen 19 unterschiedlichen Durchmessers versehen werden.

Ein weiterer Unterschied zu der Ausführungsform gemäß Fig. 3 besteht darin, dass im ersten Kanalabschnitt 21 längliche Vertiefungen 25 in der Wand des Austrittskanals 18 ausgebildet sind. Durch die Vertiefungen 25 hat der Klebstoff 24 mehr Platz, um in dem Spalt 16 nach hinten vorzudringen.

In Fig. 5 ist ein erfindungsgemäßer Abgabebehälter gezeigt, bei dem eine Kappe 26 auf die Ausbringspitze 17 aufgesteckt ist. Durch die Kappe 26 wird die Dentalmasse vor Kontakt mit der Umgebungsluft und die Kanüle 19 vor Stoßbeschädigungen geschützt. Um das Aufstecken und Abziehen der Kappe 26 zu erleichtern, sind auf der Außenseite der Kappe 26 Längsrippen ausgebildet, die in der Schnittdarstellung der Fig. 6 nicht sichtbar sind. Die Kanüle 19 ist an ihrer vorderen Stirnfläche geschlossen und hat stattdessen Austrittsöffnungen 30, die zur Seite weisen.

Auf der Innenwand der Kappe 26 sind umlaufende Stege 27 ausgebildet, die an den Umfang der Ausbringspitze 17 angepasst sind. Die Stege 27 liegen auf der Ausbringspitze 17 auf, wodurch die Kappe 26 gehalten wird und eine Abdichtung gegenüber der Umgebung stattfindet.

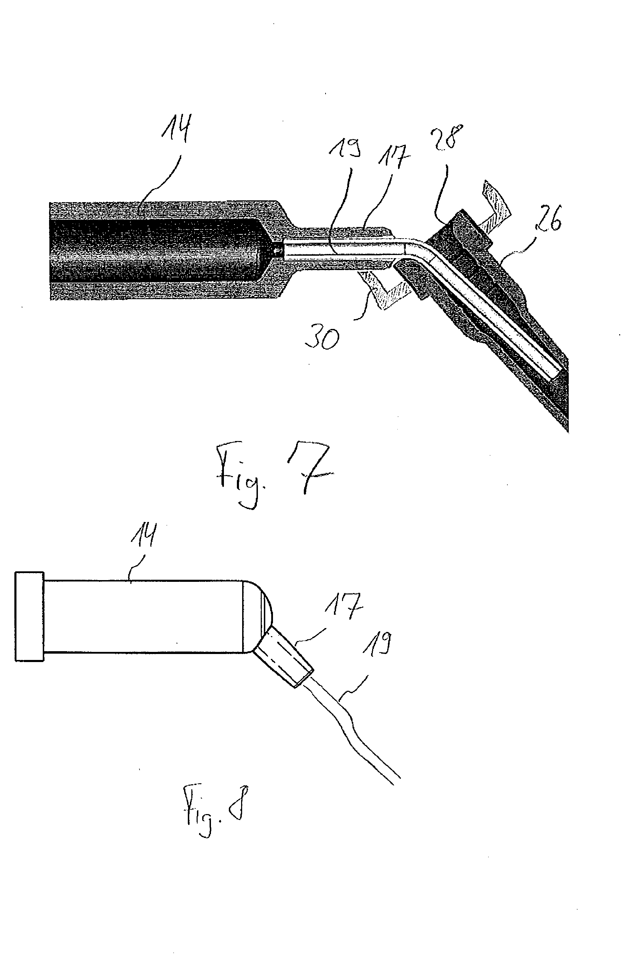

Die Kappe 26 ist an ihrem hinteren Ende mit einer nach außen weisenden Wölbung 28 versehen. Die Wölbung 28 kann gemäß Fig. 7 als Widerlager verwendet werden, um die Kanüle 19 mit Hilfe der Kappe 26 umzubiegen. Die Kappe 26 umfasst einen Anschlag 30 an ihrem hinteren Ende. Der Anschlag 30 schlägt dann an der Ausbringspitze 17 an, wenn die Kanüle 19 im richtigen Winkel - im dargestellten Beispiel etwa 30° - umgebogen ist.

In der Ausführungsform der Fig. 8 erstreckt sich die Ausbringspitze 17 unter einem Winkel von etwa 40° relativ zur Längsachse des Gehäuses 14. Bei dieser Gestaltung ist es nicht erforderlich, die Kanüle 19 umzubiegen, bevor sie in die Kavität eines Backenzahns eingeführt werden kann. Die Kanüle 19 besteht aus elastischem Polypropylen, so dass die Kanüle 19 beim Einführen flexibel dem Verlauf des Wurzelkanals folgen kann.

## Patentansprüche

1. Abgabesystem zur Abgabe von Dentalmasse, umfassend einen Abgabebehälter, einen Kolben (29) und ein Ausbringwerkzeug mit einem Stößel zur Ausübung von Druck auf den Kolben (29), wobei der Abgabebehälter ein Gehäuse (14) aufweist, an dessen Innenseite eine Gleitfläche (32) ausgebildet ist, die zum Zusammenwirken mit dem Kolben (29) bestimmt ist, wobei das Gehäuse (14) einen Innenraum umschließt und mit einem Austrittskanal (18) versehen ist, wobei in den Austrittskanal (18) ein Abschnitt einer Kanüle (19) eingeführt ist, und wobei das Gehäuse (14) mit Austrittskanal (18) als einstückiges Spritzgussteil ausgeführt ist, wobei zwischen dem Umfang der Kanüle (19) und dem Austrittskanal (18) ein Spalt (16) besteht und wobei in den Spalt ein Klebstoff (24) eingebracht ist.

2. Abgabesystem nach Anspruch 1, **dadurch gekennzeichnet, dass** in dem Austrittskanal (18) ein Anschlag (23) für die Kanüle (19) vorgesehen ist.

3. Abgabesystem nach Anspruch 1 oder 2, **dadurch gekennzeichnet, dass** der Austrittskanal (18) eine Seitenführung für die Kanüle (19) bietet.

4. Abgabesystem nach Anspruch 3, **dadurch gekennzeichnet, dass** der Austrittskanal (18) einen ersten Kanalabschnitt (20) und einen zweiten Kanalabschnitt (21) umfasst und dass im ersten Kanalabschnitt (20) die Innenfläche des Austrittskanals (18) an die Außenfläche der Kanüle (19) angepasst ist.

5. Abgabesystem nach einem der Ansprüche 1 bis 4, **dadurch gekennzeichnet, dass** der Austrittskanal (18) sich in einer Richtung verjüngt, die entgegengesetzt zu der Richtung ist, in der die Kanüle (19) aus dem Austrittskanal (18) herausragt.

6. Abgabesystem nach einem der Ansprüche 1 bis 5, **dadurch gekennzeichnet, dass** der Austrittskanal (18) eine Mündungsaufweitung (22) aufweist.

7. Abgabesystem nach einem der Ansprüche 1 bis 6, **dadurch gekennzeichnet, dass** die Kanüle (19) biegbar ist.

8. Abgabesystem nach einem der Ansprüche 1 bis 7, **dadurch gekennzeichnet, dass** das Gehäuse (14) eine Ausbringspitze (17) umfasst, durch die sich der Austrittkanal (18) hindurch erstreckt, und dass eine an die Ausbringspitze (17) angepasste Kappe (26) umfasst ist.

9. Abgabesystem nach Anspruch 8, **dadurch gekennzeichnet, dass** die Innenwand der Kappe (26) an einem Ende, das der Richtung, in der die Kanüle (19) aus dem Austrittskanal (18) herausragt, entgegengesetzt ist, eine nach außen gerichtete Wölbung (28) aufweist.

10. Abgabesystem nach Anspruch 8 oder 9, **dadurch gekennzeichnet, dass** die Kappe (26) mit einem Anschlag (30) versehen ist und dass der Anschlag an dem Gehäuse (14) anliegt, wenn die Kanüle (19) um einen vorgegebenen Winkel umgebogen ist.

11. Abgabesystem nach einem der Ansprüche 8 bis 10, **dadurch gekennzeichnet, dass** auf der Innenwand der Kappe (26) ein umlaufender Steg (27) ausgebildet ist.

12. Abgabesystem nach einem der Ansprüche 1 bis 11, **dadurch gekennzeichnet, dass** der Austrittskanal (18) mit der Längsachse des Gehäuses (14) einen Winkel einschließt, wobei der Winkel vorzugsweise zwischen 25° und 60°, weiter vorzugsweise zwischen 30° und 45° liegt.

13. Abgabesystem nach einem der Ansprüche 1 bis 12, **dadurch gekennzeichnet, dass** die Kanüle (19) eine elastische Flexibilität aufweist.

## Claims

1. Dispensing system for dispensing dental compound, said dispensing system comprising a dispensing container, a plunger (29) and a discharge tool with a ram for applying pressure to the plunger (29), the dispensing container having a housing (14), on the inside of which a slide surface (32) is formed that is intended for engagement with the plunger (29), the housing (14) enclosing an interior and being provided with an outlet channel (18), a portion of a needle (19) being inserted into the outlet channel (18), and the housing (14) with outlet channel (18) being designed as a one-piece injection-molded part, a gap (16) being present between the circumference of the needle (19) and the outlet channel (18), and an adhesive (24) being introduced into the gap.

2. Dispensing system according to Claim 1, **characterized in that** a limit stop (23) for the needle (19) is provided in the outlet channel (18).

3. Dispensing system according to Claim 1 or 2, **characterized in that** the outlet channel (18) provides a lateral guide for the needle (19).

4. Dispensing system according to Claim 3, **characterized in that** the outlet channel (18) comprises a first channel portion (20) and a second channel portion (21), and **in that** the inner surface of the outlet channel (18) is adapted to the outer surface of the needle (19) in the first channel portion (20).

5. Dispensing system according to one of Claims 1 to 4, **characterized in that** the outlet channel (18) narrows in a direction opposite the direction in which the needle (19) protrudes from the outlet channel (18).

6. Dispensing system according to one of Claims 1 to 5, **characterized in that** the outlet channel (18) has a widening mouth (22).

7. Dispensing system according to one of Claims 1 to 6, **characterized in that** the needle (19) is bendable.

8. Dispensing system according to one of Claims 1 to 7, **characterized in that** the housing (14) comprises a discharge tip (17) through which the outlet channel (18) extends, and **in that** a cap (26) adapted to the discharge tip (17) is provided.

9. Dispensing system according to Claim 8, **characterized in that** the inner wall of the cap (26) has an outwardly directed bulge (28) at an end opposite the direction in which the needle (19) protrudes from the outlet channel (18).

10. Dispensing system according to Claim 8 or 9, **characterized in that** the cap (26) is provided with a limit stop (30), and **in that** the limit stop bears on the housing (14) when the needle (19) is bent through a predetermined angle.

11. Dispensing system according to one of Claims 8 to 10, **characterized in that** a peripheral web (27) is formed on the inner wall of the cap (26).

12. Dispensing system according to one of Claims 1 to 11, **characterized in that** the outlet channel (18) encloses an angle with the longitudinal axis of the housing (14), said angle preferably being between 25° and 60°, more preferably between 30° and 45°.

13. Dispensing system according to one of Claims 1 to 12, **characterized in that** the needle (19) has an elastic flexibility.

## Revendications

1. Système de distribution pour la distribution de masse dentaire, comprenant un récipient de distribution, un piston (29) et un outil d'expulsion ayant un poussoir pour l'exercice d'une pression sur le piston (29), le récipient de distribution présentant un logement (14) sur le côté intérieur duquel est réalisée une surface de glissement (32) qui est destinée à coopérer avec le piston (29), le logement (14) comprenant un espace intérieur et étant doté d'un canal de sortie (18), une section d'une canule (19) étant insérée dans le canal de sortie (18) et le logement (14) étant réalisé comme une seule pièce moulée par injection avec le canal de sortie (18), étant entendu qu'il existe un interstice (16) entre le périmètre de la canule (19) et le canal de sortie (18) et qu'un adhésif (24) est introduit dans cet interstice.

2. Système de distribution selon la revendication 1, **caractérisé en ce qu'**une butée (23) est prévue pour la canule (19) dans le canal de sortie (18).

3. Système de distribution selon la revendication 1 ou 2, **caractérisé en ce que** le canal de sortie (18) offre un guidage latéral pour la canule (19).

4. Système de distribution selon la revendication 3, **caractérisé en ce que** le canal de sortie (18) comprend une première section de canal (20) et une deuxième section de canal (21) et **en ce que** dans la première section de canal (20), la surface intérieure du canal de sortie (18) est adaptée à la surface extérieure de la canule (19).

5. Système de distribution selon l'une des revendications 1 à 4, **caractérisé en ce que** le canal de sortie (18) se rétrécit dans une direction qui est opposée à la direction dans laquelle la canule (19) fait saillie en dehors du canal de sortie (18).

6. Système de distribution selon l'une des revendications 1 à 5, **caractérisé en ce que** le canal de sortie (18) présente un élargissement de son embouchure (22).

7. Système de distribution selon l'une des revendications 1 à 6, **caractérisé en ce que** la canule (19) peut être pliée.

8. Système de distribution selon l'une des revendications 1 à 7, **caractérisé en ce que** le logement (14) comprend une pointe d'expulsion (17) à travers laquelle s'étend le canal de sortie (18) et **en ce qu'**un bouchon (26) adapté à la pointe d'expulsion (17) est prévu.

9. Système de distribution selon la revendication 8, **caractérisé en ce que** la paroi intérieure du bouchon (26) présente, au niveau d'une extrémité qui est opposée à la direction dans laquelle la canule (19) fait saillie en dehors du canal de sortie (18), une courbure (28) dirigée vers l'extérieur.

10. Système de distribution selon la revendication 8 ou 9, **caractérisé en ce que** le bouchon (26) est muni d'une butée (30) et **en ce que** la butée prend appui sur le logement (14) lorsque la canule (19) est pliée dans un angle prédéfini.

11. Système de distribution selon l'une des revendications 8 à 10, **caractérisé en ce qu'**une nervure périphérique (27) est réalisée sur la paroi intérieure du bouchon (26).

12. Système de distribution selon l'une des revendications 1 à 11, **caractérisé en ce que** le canal de sortie (18) inclut un angle avec l'axe longitudinal du logement (14), cet angle étant compris de façon préférée entre 25° et 60°, et de façon plus préférée entre 30° et 45°.

13. Système de distribution selon l'une des revendications 1 à 12, **caractérisé en ce que** la canule (19) présente une flexibilité élastique.
